# EUROPEAN PATENT SPECIFICATION

(11) **EP 3 226 745 B1**
(45) Date of publication and mention of the grant of the patent: **17.03.2021**
(21) Application number: 14907352.0
(22) Date of filing: 04.12.2014
(51) Int. Cl.: A61B 1/00, A61B 1/04, A61B 1/045, A61P 1/00

(54) **CAPSULE COATING FOR IMAGE CAPTURE CONTROL**
KAPSELBESCHICHTUNG ZUR BILDAUFNAHMESTEUERUNG
ENROBAGE DE CAPSULE POUR COMMANDE DE CAPTURE D'IMAGE

(43) Date of publication of application: 11.10.2017
(73) Proprietor: CapsoVision, Inc., Saratoga, CA 95070 (US); Trollsas, Mikael, San Jose, CA 95124 (US); Hadley, Mark, Newark, CA 94560 (US)
(72) Inventor: TROLLSAS, Mikael, San Jose, California 95124 (US); HADLEY, Mark, Newark, California 94560 (US)
(74) Representative: Papula Oy
(86) International application number: PCT/US2014/068601
(87) International publication number: WO 2016/089413

(56) References cited:
- US-A1- 2003 040 685
- US-A1- 2006 178 557
- US-A1- 2008 143 822
- US-A1- 2010 021 536
- US-A1- 2010 324 381
- US-A1- 2011 306 855
- US-A1- 2013 137 993

## Description

### CROSS REFERENCE TO RELATED APPLICATIONS

The present disclosure is related to U.S. Patent No. 7,983,458, entitled "in vivo Autonomous Camera with On-Board Data Storage or Digital Wireless Transmission in Regulatory Approved Band", granted on July 19, 2011 and U.S. Patent Application No. 13/762,153, entitled "Self Assembly of In-Vivo Capsule System", filed on Feb. 7, 2013.

### FIELD OF THE INVENTION

The present invention relates to diagnostic imaging inside the human body or any other living creature. In particular, the present invention relates to an in-vivo capsule that uses an enteric coating on the outer surface of the capsule housing and detects whether the coating has dissolved as a means to control image capture.

### BACKGROUND AND RELATED ART

Devices for imaging body cavities or passages *in vivo* are known in the art and include endoscopes and autonomous encapsulated cameras. Endoscopes are flexible or rigid tubes that pass into the body through an orifice or surgical opening, typically into the esophagus via the mouth or into the colon via the rectum. An image is formed at the distal end using a lens and transmitted to the proximal end, outside the body, either by a lens-relay system or by a coherent fiber-optic bundle. A conceptually similar instrument might record an image electronically at the distal end, for example using a CCD or CMOS sensor array, and transfer the image data as an electrical signal to the proximal end through a cable. Endoscopes allow a physician or a veterinary physician control over the field of view and are well-accepted diagnostic tools. However, they do have a number of limitations, present risks to the patient, are invasive and uncomfortable for the patient, and their cost restricts their application as routine health-screening tools.

Because of the difficulty traversing a convoluted passage, endoscopes cannot easily reach the majority of the small intestine and special techniques and precautions, that add cost, are required to reach the entirety of the colon. Endoscopic risks include the possible perforation of the bodily organs traversed and complications arising from anesthesia. Moreover, a trade-off must be made between patient pain during the procedure and the health risks and post-procedural down time associated with anesthesia.

An alternative *in vivo* image sensor that addresses many of these problems is the capsule endoscope. A camera is housed in a swallowable capsule, along with a radio transmitter for transmitting data, primarily comprising images recorded by the digital camera, to a base-station receiver or transceiver and data recorder outside the body. The capsule may also include a radio receiver for receiving instructions or other data from a base-station transmitter. Instead of radio-frequency transmission, lower-frequency electromagnetic signals may be used. Power may be supplied inductively from an external inductor to an internal inductor within the capsule or from a battery within the capsule.

An autonomous capsule camera system with on-board data storage was disclosed in the US Patent No. 7,983,458, entitled "In Vivo Autonomous Camera with On-Board Data Storage or Digital Wireless Transmission in Regulatory Approved Band," granted on July 19, 2011. This patent describes a capsule system using on-board storage such as semiconductor nonvolatile archival memory to store captured images. After the capsule passes from the body, it is retrieved. Capsule housing is opened and the images stored are transferred to a computer workstation for storage and analysis. For capsule images either received through wireless transmission or retrieved from on-board storage, the images will have to be displayed and examined by diagnostician to identify potential anomalies. Prior art further includes US 2011/306855 A1 which discloses detection of bleeding in-vivo. Detected signals are compared with a predetermined threshold calculated from transmission spectra of bile and of blood, and the presence and/or concentration of bile and blood in-vivo is determined.

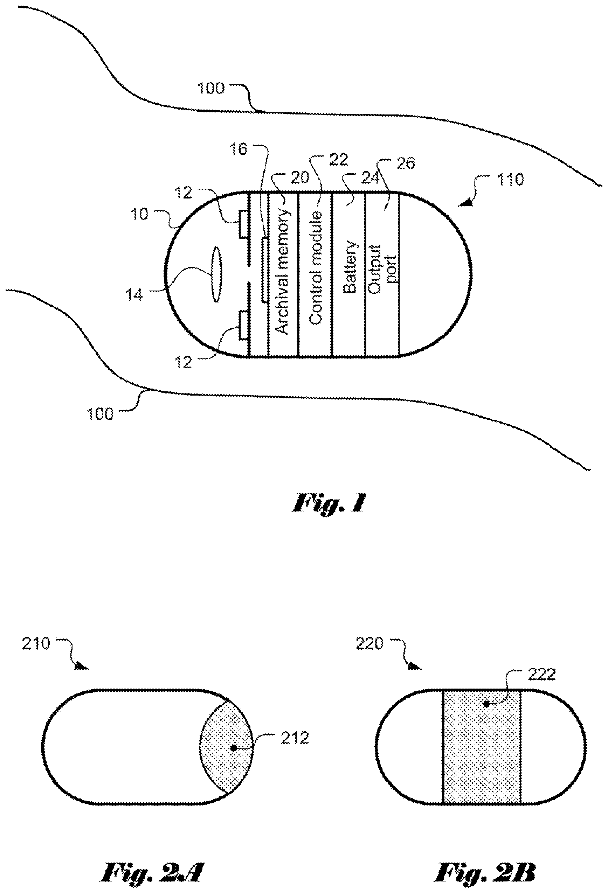

Fig. 1 illustrates an exemplary capsule system with on-board storage. The capsule device 110 includes illuminating system 12 and a camera that includes optical system 14 and image sensor 16. A semiconductor nonvolatile archival memory 20 may be provided to allow the images to be stored and later retrieved at a docking station outside the body, after the capsule is recovered. Capsule device 110 includes battery power supply 24 and an output port 26. Capsule device 110 may be propelled through the gastrointestinal (GI) tract by peristalsis.

Illuminating system 12 may be implemented by LEDs. In Fig. 1, the LEDs are located adjacent to the camera's aperture, although other configurations are possible. The light source may also be provided, for example, behind the aperture. Other light sources, such as laser diodes, may also be used. Alternatively, white light sources or a combination of two or more narrow-wavelength-band sources may also be used. White LEDs are available that may include a blue LED or a violet LED, along with phosphorescent materials that are excited by the LED light to emit light at longer wavelengths. The portion of capsule housing 10 that allows light to pass through may be made from bio-compatible glass or polymer.

Optical system 14, which may include multiple refractive, diffractive, or reflective lens elements, provides an image of the lumen walls (100) on image sensor 16. Image sensor 16 may be provided by charged-coupled devices (CCD) or complementary metal-oxide-semiconductor (CMOS) type devices that convert the received light intensities into corresponding electrical signals. Image sensor 16 may have a monochromatic response or include a color filter array such that a color image may be captured (e.g. using the RGB or CYM representations). The analog signals from image sensor 16 are preferably converted into digital form to allow processing in digital form. Such conversion may be accomplished using an analog-to-digital (A/D) converter, which may be provided inside the sensor (as in the current case), or in another portion inside capsule housing 10. The A/D unit may be provided between image sensor 16 and the rest of the system. LEDs in illuminating system 12 are synchronized with the operations of image sensor 16. Processing module 22 may be used to provide processing required for the system such as image processing and video compression. The processing module may also provide needed system control such as to control the LEDs during image capture operation. The processing module may also be responsible for other functions such as managing image capture and coordinating image retrieval.

After the capsule camera traveled through the GI tract and exits from the body, the capsule camera is retrieved and the images stored in the archival memory are read out through the output port. The received images are usually transferred to a base station for processing and for a diagnostician to examine. The accuracy as well as efficiency of diagnostics is most important. A diagnostician is expected to examine the images and correctly identify any anomaly.

When the capsule device travels through the GI tract, the capsule device will encounter different environments. It is desirable to manage the capsule device to travel at a speed that sufficient sensor data (e.g., images) can be collected at all locations along the portions of the GI tract which are of interest, without wasting battery power and/or data storage by collecting excessive data in some locations. In order to manage the capsule device to travel at a relatively steady speed, techniques have been developed to change the capsule specific gravity during the course of travelling through the GI tract. In some environments, it is desirable to have a capsule with higher specific gravity. In other environments, it may be desirable to have a capsule with lower specific gravity. For example, it is desirable to configure the capsule device to have a lower specific gravity when the capsule device travels through the ascending colon. On the other hand, it may be desirable to configure the capsule device to have a higher specific gravity when the capsule device travels through the descending colon if the descending colon is filled with liquid. However, techniques based on specific gravity or density control may not work reliably due to various reasons. For example, the change of specific gravity or density may not have to take place at the intended section of the GI tract. Therefore, the location of the capsule device inside the GI tract has to be monitored or estimated. However, the location of the capsule device usually cannot be accurately determined without the use of additional equipment outside the patient's body. Therefore, it is desirable to develop reliable means to manage the capsule device to travel at a relatively steady speed in the GI tract.

The capsule device is operated by battery when it travels inside the human body. For autonomous capsule endoscope, the capsule device has to capture a large amount of images, typically in the order of tens of thousands or more during the course of travelling through the human body. In addition, the capsule may also include other sensors (for example, PH meter and ultrasonic sensor) to capture other sensory data. It is very crucial to tightly manage the power consumption of the capsule device so that the capsule device will consume power only when needed. Consequently, the capsule device will be able to perform successful image/sensory data capture.

### BRIEF SUMMARY OF THE INVENTION

The present invention is defined by the capsule device of independent claim 9 and a corresponding method of image capture control of claim 1 for the capsule device, where in the capsule device remains in a very low power state for monitoring purpose (i.e., monitoring mode) after it is administered into a human body through the mouth. The capsule device is coated with an enteric material that is expected to dissolve after it enters the small bowel. In order to determine when the capsule device enters the small bowel, the capsule device is configured to operate at a low-power monitor mode. During this monitoring state, the capsule device captures a first image using the camera and the light source at a first frame rate substantially below a target frame rate after the capsule device is administered into a human body. The capsule device compares first information related to the first image with second information related to a coating image corresponding to the coating. If the first information matches the second information, the capsule device remains in the monitoring state by repeating capturing the first image and comparing the first information related to the first image with the second information related to the coating image. If the first information does not matches the second information, the capsule device declares that the coating has dissolved and the capsule device configures the camera to capture third images at the target frame rate after declaring that the coating has dissolved. The process of comparing the first information with the second information can be performed inside or outside the capsule device. In the case that the task is performed outside the capsule device, the capsule device also includes a wireless transmitter to transmit the first images to a wireless receiver outside the capsule device.

The first/second information to be compared may be related to the color, brightness or contents of the corresponding images. The first/second information can be derived based on image resolution, image size, or both. The first images may be captured with reduce luminous energy to conserve energy during the monitoring mode. The coating may contain an area with a pre-defined color or brightness. The first/second information comparison may be based on the average or median value of the color or the brightness of the underlying images respectively. The coating may contain an area with a pre-defined pattern. Image matching between the first images and the coating image can be performed based on selected samples of respective images. To further simplify the comparing process, the samples can be converted to a binary data so that simple logic comparison can be used and the number of matched samples can be easily counted and used to make decision regarding whether the coating has been dissolved. If the coating is determined to have dissolved, the capsule device can be configured to capture images at a desired frame rate. Alternatively, the capsule device can be configured to wait for a period of time to ensure that the coating has dissolved completely before starting capturing images at a desired frame rate.

The coating image used for comparison can be obtained off-line using a sample capsule in an environment similar to the targeted use (i.e., inside the gastrointestinal track). In this case, the second information can be determined off-line and stored in each capsule device. The coating image may also be captured using the capsule device during the period of time that the coating is not dissolved. For example, this can be done shortly after the capsule device is swallowed.

### BRIEF DESCRIPTION OF THE DRAWINGS

Fig. 1 shows schematically a capsule camera system in the GI tract, where archival memory is used to store captured images to be analyzed and/or examined.
Fig. 2A - Fig 2B illustrate examples of a capsule device incorporating an enteric coating to cover at least the field of view of the camera, where the enteric coating for the capsule device with a forward-looking camera is shown in Fig. 2A and the enteric coating for the capsule device with a panoramic camera around the mid-body of the capsule device is shown in Fig. 2B.
Fig. 3 illustrates an exemplary flowchart of capture control for a capsule device with an enteric coating according to an embodiment.

### DETAILED DESCRIPTION OF THE DISCLOSURE

It will be readily understood that the components of the present disclosure, as generally described and illustrated in the figures herein, may be arranged and designed in a wide variety of different configurations. Thus, the following more detailed description of the embodiments of the systems and methods of the present disclosure, as represented in the figures, is not intended to limit the scope of the invention, as claimed, but is merely representative of selected embodiments of the disclosure.

Reference throughout this specification to "one embodiment," "an embodiment," or similar language means that a particular feature, structure, or characteristic described in connection with the embodiment may be included in at least one embodiment of the present disclosure. Thus, appearances of the phrases "in one embodiment" or "in an embodiment" in various places throughout this specification are not necessarily all referring to the same embodiment.

Furthermore, the described features, structures, or characteristics may be combined in any suitable manner in one or more embodiments. One skilled in the relevant art will recognize, however, that the disclosure can be practiced without one or more of the specific details, or with other methods, components, etc. In other instances, well-known structures, or operations are not shown or described in detail to avoid obscuring aspects of the disclosure.

The illustrated embodiments of the disclosure will be best understood by reference to the drawings, wherein like parts are designated by like numerals throughout. The following description is intended only by way of example, and simply illustrates certain selected embodiments of apparatus and methods that are consistent with the invention as claimed herein.

In some applications, it is desirable to operate the capsule in an active capture mode to image a designated section of the gastrointestinal. For example, it may be desirable to image only the small bowel and colon of the GI track. Therefore it is desirable to set the capsule into the active image capture mode when it enters the small bowel. In other words, the capsule is set to an inactive mode before it enters the small bowel. In U.S. Patent No. 8,187,174, entitled "Detection of when a capsule camera enters into or goes out of human body and associated operations", issued on May 29, 2012, a technique to distinguish whether a capsule is inside or outside the human body is disclosed. However, the technique disclosed in U.S. Patent No. 8,187,174 does not provide a reliable way to further distinguish whether the capsule enters the small bowel or not.

In U.S. Patent Application No. 13/762,153, entitled "Self Assembly of In-Vivo Capsule System", filed on Feb. 7, 2013, a capsule with a coating or outer housing is disclosed where the coating or the outer housing dissolves in the GI tract. The capsule device has a connector encapsulated by the coating or outer housing. The coating or outer housing may be made of materials to break down in the small bowel or colon rather than the stomach. Therefore, the connector may become exposed when the capsule travels to the small bowel or colon. The capsule may be attached to another capsule when these two capsules become in contact. The joined capsules may achieve a specific gravity as desired.

The present disclosure discloses a means that utilizes a coating on the outer surface of the capsule to determine whether the capsule enters a targeted part of the gastrointestinal (GI) track. A type of coating materials is selected for a target part of the GI tract (e.g., the duodenum, jejunum, ileum, cecum or the colon), so that the coating is expected to dissolve when the capsule enters the targeted part or within a small time window when the capsule enters the targeted part. When the coating is present, the camera of the capsule device will capture a coating image. On the other hand, when the coating has dissolved, the camera will be able to capture an image of the lumen wall in the field view of the camera. Therefore, the capsule may use image processing techniques to determine whether the coating has dissolved. Upon determining whether the coating has dissolved, which implies whether the capsule has enters a targeted part of GI track, the capsule may be configured accordingly to perform desired tasks such as to enter active image capture mode.

In one embodiment of the present disclosure, an enteric coating is applied on the outer surface of the capsule. This coating applied to the outer surface of the capsule housing should be easily distinguishable from the gastrointestinal (GI) tract by making the color, brightness, contrast and/or contents of the coating image to be such that is not commonly found in the GI tract. This coating can be engineered to dissolve in a target section of the GI tract, i.e., the small bowel in this example. When the coating is still present, the images captured by the capsule camera will be very different from that without the coating. Therefore, the system can determine if the coating is still present or has dissolved. Once the coating has dissolved, the system can detect this case based on images captured. Accordingly, the system can be configured to start taking and storing or transmitting images of the intestine at the desired rate.

One of the main purposes of the coating is to allow the system to easily determine whether the capsule has entered the small bowel. The system should be configured as an energy conservation mode to operate at a level of power consumption as low as possible before it enters the small bowel. In the energy conservation mode, the system needs to perform the task of monitoring whether it has entered the small bowel by detecting whether the coating is still present or not. However, the task of monitoring whether it has entered the small bowel will consume power. Consequently, this task has to be performed using relatively low level of power. For example, the task can be performed infrequently such as at one hundredth of the frame rate of a desired frame rate or lower. In practice, any frame rate substantially below the regular frame capture rate can be used for detecting coating presence. One factor related to the proper frequency to perform the task of determining whether it has entered the small bowel may depend on the period of time required for the coating to substantially dissolve. The period between each checking the coating status may correspond to a fraction of the expected time for the coating to dissolve. Other factors, such as the expected travel speed of the capsule within the GI track, may also be taken into consideration to determine the proper frequency to perform the task.

Other means to perform the task in low level of power consumption can also be used. For example, the image captured for the purpose of determining whether the capsule enters the small bowel can be performed with reduced luminous energy from the internal light sources inside the capsule. The images can be captured with reduced light level as long as the light level is sufficient to discern whether the captured image corresponds to a reflection from the coating or a real image of the GI track. If reduced luminous energy is used during the monitoring mode, the coating image used for comparing with a currently captured image has to be captured with reduce luminous energy as well. An image with reduced resolution or image size may also be used to determine whether the capsule enters the small bowel. For example, if the outer surface of the housing is coated with a blue or green material, the captured image will correspond to a roughly uniform blue or green field if the coating is still present. Therefore, a small number of samples from the captured image will be sufficient to derive average color reasonably accurate. The derived average color can be used to determine whether the capsule has enters small bowel. Beside the color, other image attributes such as brightness or contract may be used as well. If contract is used, the coating image should include at least two areas with two corresponding intensity levels. The differences between the two intensity levels can be used as an indication regarding whether the coating has dissolved.

The various low-level means to perform the task of determining whether the capsule has entered the small bowel can be combined to further reduce the power consumption. For example, the task can be performed at one hundredth of the desired frame rate for the coating to dissolve; the image can be captured at one quarter of light level of regular image capture; and only 16 distributed samples may be used to determine whether the capsule device has entered the small bowel.

The advantage of a coated capsule according to the present disclosure is that while the coating is on the capsule, the capsule can be configured to operate at a very low power consumption mode, such as a very slow frame rate so that the capsule device only need to capture images with very low-level processing and determine if the coating has dissolved or not. This low power state will allow the battery or batteries in the capsule to last much longer than compared to a system that begins taking images immediately after swallowing at the desired frame rate continuously. In addition to battery life, a capsule device with on-board archival memory operated according to the present disclosure will be able to spare the need to store the images corresponding to the unintended areas. Consequently, the capsule device will have more efficient memory usage and capture more intended images.

Depending on the configuration of the camera or cameras in the capsule, an area of the coating at one end in the longitudinal direction may be imaged (a forward-looking camera configuration) or a circular area around the longitudinal axis of the capsule may be imaged (an all-around panoramic camera configuration). An embodiment of the present disclosure disposes a coating on the housing to cover at least an area to be imaged. Fig. 2A illustrates an example of a coating (212) for a capsule device (210) with forward-looking camera arrangement. The area 212 is in the field of field for the camera. It may cover a partial area for the field of view. In this case, only the area of the captured image corresponding to the area covered by the coating needs to be used for monitoring whether the coating has dissolved. In the case that the capsule device (220) uses a panoramic camera having a field of view around the circular area in the middle part of the capsule, the coating (222) can be deposed on the corresponding circular area as shown in Fig. 2B. The coating may also cover the entire capsule housing for convenience.

The coating can be engineered to dissolve within a target period when the capsule enters a target part of the GI track. In the case of enteric coating, the coating may dissolve within a period of time after the pH of the intestine has risen from the acidic state in the stomach to the more neutral or slightly alkaline pH of the small bowel. In order to determine whether the coating has dissolved, the capsule has to compare a newly capture with a prior picture or a reference picture. In one embodiment, picture color is used for comparing a newly captured picture against a prior picture or a reference picture. In this case, when the capsule is first swallowed, it takes an image at a very slow frame rate (e.g., every 5 minutes) and then compares the average color of a number of the pixels in the area of the coating to the known coating color. Initially this comparison will indicate a good match with the known color, but once the coating is dissolved, the comparison will show more distinction from the known color. The system can determine when the difference between the color of the average of captured samples and the color of the known coating exceeds a threshold. The capsule then begins to start taking pictures at the desired frame rate or waits some period of time (if desired) before switching to the active capture mode.

In another embodiment of the present disclosure, the coating may correspond to a pre-defined pattern. The captured image of this pre-defined pattern can be easily detected using matching techniques. For example, a checker board pattern may be used and the pattern can be deposed on the outer surface of the housing with a fixed relative location to the sensor. Therefore, the captured image will correspond to a known pre-defined image. The captured image can be matched with the known pre-defined image to determine whether the coating is present or not. The image matching can be performed at substantially reduced resolution to conserve system power. For example, a small number of samples from each patch of the checker board pattern may be used for image matching. The average or mean value of the small number of samples from each patch of the checker board pattern may be determined. The average or mean value can be further represented as a binary value, such as a value of 1 for the white patch and a value of 0 for the block patch. Therefore, by simply counting the number of matched samples (i.e., number of matched patches in this case) can provide a good indication regarding whether the captured image corresponds to the coating with pre-defined pattern. If so, it is determined that the coating has not yet dissolved. Otherwise, it is determined that the coating has dissolved. Other patterns may also be used. For example, alternative black and white lines may be used. In another example, multi-level patches may also be used, such as 4 gray levels corresponding to 0, 80, 160 and 240 may be used for an 8-bit image data.

The coating image may be generated *in vivo* after the capsule device is swallowed. While it may take a capsule device different time to travel to the small bowel after it is swallowed, it is almost certain that the coating will stay on for the first 30 minutes. Therefore, the coating image may be generated during this period. To further reduce power, the capsule device may be placed in a sleep mode that only counts the time to wake up. Near the end of the period, the capsule device is waken up to capture the coating image and to derive information associated with the coating image for comparison with the information derived based on subsequently captured image. The subsequently captured images may still be the same as the coating image (i.e., coating on) or new images of the GI track wall (i.e., coating dissolved). Alternatively, the coating image can be generated off-line in an environment similar to that inside the GI tract. In this case, a sample capsule device with the same type of camera, the same type of light source and the same type coating can be used to generate the sample coating image, also referred as a *reference* coating image in this disclosure. The reference coating image should be taken with the same settings of light source and camera setting. The reference coating image can be loaded into memory inside the capsule before it is administered into a human body. Therefore, an image captured by the capsule device can be compared with this reference coating image to determine whether the coating is still present or not. While the reference coating image can be stored in the capsule device, parameters derived from the reference image may be stored and used for comparing with a captured image to determine whether the coating has dissolved or not. For example, if a checker board pattern is used, the parameters may include the size of the patch and the pixel values of the two patches. Furthermore, the pixel values of the two patches may be represented by two binary values (i.e., 0 and 1). In this case, the parameters corresponding to the reference coating image can be very compact. Therefore, there is no need to generate coating image or to derive the associate information by each capsule device. This approach should work reliably if the manufacturing variations among individual capsule cameras are small.

As mentioned before, specific coating materials may be used for intended targeted part of the GI track. For example, enteric coating does not dissolve in the low pH (acidic) of the stomach (typically between 1.5 to 3.5). However, the coating does dissolve when the pH becomes more neutral. Therefore, enteric coating may be used if the targeted part is after the stomach such as the duodenum (typically pH around 5-6), jejunum (typically pH between 7-8), ileum (typically pH between 7-8), cecum (pH around 6) or colon (typically between 5.5 and 7, slightly acid to neutral).

Depending on the desired targeted part, various materials can be selected for the enteric coating. General enteric coatings may correspond to carboxymethylcellulose (CMC), acid substituted cellulose, diacid substituted cellulose such as phthalate and succinate but not limited to those, triacid substituted cellulose such as trimellitic acid and citric acid (CA), acid substituted poly(vinyl alcohol or acetate) (PVA/PVAP), acid-, diacid-, or triacid substituted hydroxypropyl methylcellulose such as phthalic-, succinic-, trimellitic-, and citric- but not limited to those, various substituted methacrylic- and acrylic acid copolymers , fatty acids, waxes, shellac (esters of aleuritic acid (pH 7.0), plastics, and plant fiber . Any of the above polymers with a Tg> 45°C, Tg>75°C, Tg> 120°C,Tg> 130°C, Tg> 135°C, or Tg> 140°C, Tg corresponds to Glass Transition Temperature. Examples of the enteric coatings include carboxymethylcellulose (CMC) (solubility > pH 5.0), acetate phthalate (CAP) (solubility > pH 6.2), cellulose acetate trimellitate (CAT) (pH 5.0), poly(vinyl acetate phthalate) (PVAP) (> pH 5.2), hydroxypropyl methylcellulose phthalate (HPMCP) (> pH 4.5-5.5), methacrylic acid copolymer type C/poly(methacrylic acid-co-methyl methacrylate) (pH 5.5-7.0), fatty acids, waxes, shellac (esters of aleuritic acid) (pH 7.0), plastics, and plant fiber. The enteric materials listed are just some examples of such materials that can be used for coating. It is not intended for an exhaustive list of all possible enteric material.

While the enteric coating has its dissolvability depending on the acidity, the coating dissolvability depending on other factors of the intraluminal environment. For example, the dissolution or the degradation of the coating can be triggered by enzymes or bacteria only present in certain part of the GI track, such as the lower bowel. Examples of the enzymatic coating include encoded azo crosslinked polymers such as methacrylic acid and/or acrylic acid copolymers, carboxymethylcellulose, hydroxypropylmethylcellulose, methacryloxy azobenzene HEMA, diisocyanate crosslinked dextran. In a similar way to the pH initiated dissolution of the coating could be engineered to dissolve within a period of thime after the capsule comes in contact with the right environment. Again, when the capsule is first swallowed, it takes an image at a very slow rate such as once every 5 minutes and then compares the captured image with a reference coating image. For example, the comparison may be based on the average color of a number of the pixels in the area of the coating to the known coating color. Initially this comparison will indicate a good match between the average color of the captured image and the pre-defined color associated with the coating. Once the coating is dissolved, the comparison will indicate discernable difference between the average color of the captured image and the pre-defined color associated with the coating. The system can determine when the difference between the detected average color and the pre-defined color becomes large enough and then begin to start taking pictures at the desired frame rate. If desired, the system may also be configured to wait some period of time to make sure all coating is removed before starting pictures at the desired regular frame rate.

The time such a capsule stays within the body is quite variable. One significant source of variability is how long the capsule stays within the esophagus and stomach. The above example removes the influence of this variability because the coating only begins to dissolve once it has entered the duodenum or perhaps the jejunum. This is where the stomach acid is neutralized. If the purpose of the capsule is to image the lower GI track, than this example could provide significantly more battery life than a capsule that starts imaging immediately after swallowing or with a preset delay.

For a capsule that only has a delay, the difficulty is that the time spent in the esophagus and the stomach is quite variable especially if the capsule's specific gravity is near one. For example, in U.S. Patent Serial No. 7,192,397 and U.S. Patent Serial No. 8, 444,554, a capsule device with specific gravity about 1 is disclosed. When the capsule device has a specific gravity about 1, the device will suspend or float in the liquid in the gastrointestinal (GI) track such as in the stomach or in the colon. As disclosed in U.S. Patent No. 7,192,397 and U.S. patent No. 8, 444,554, the capsule device will be carried through the body lumen by a flow of liquid through the body lumen when the capsule device has a specific gravity about 1. However, for an in vivo capsule device, after the capsule device is swallowed by a patient, the capsule device first goes through the pharynx and esophagus into the stomach and the stomach may be filled with liquid. The delay cannot be longer than the minimum time the capsule stays in this area or the capsule will start imaging too for the desired area of the GI tract. This disclosure removes this variability.

The coating may not necessarily be related to pH or enzyme. For example, the coating material may just slow the dissolution of the gel capsule to cause a timed coating so that the coating is expected to dissolve within a period of time. The timed coating may have double layer structures with inner layer being ionic and outer layer being any of the above enteric coatings. Water diffuses through the enteric coating into the ionic coating, which absorbs the water and once a sufficient amount of water has been absorbed by the inner layer the outer coating cracks which allow the whole coating matrix to come off. The coating include hydrophobic surfactant, water-soluble polymer, and hydroxypropyl methylcellulose (HPMC).

For completely coated capsules, this disclosure may also have a first layer of a very hydrophilic coating with poor adhesion to the capsule material. The first layer may then be protected by an enteric coating engineered to dissolve in thirty minutes to 12 hours after the pH of the intestine has risen from the acidic state in the stomach to the more neutral or slightly alkaline pH of the small bowel. The advantage of this system is to make sure all coating is easily removed from the capsule to make sure imaging quality will not be affected by any potential remaining coating.

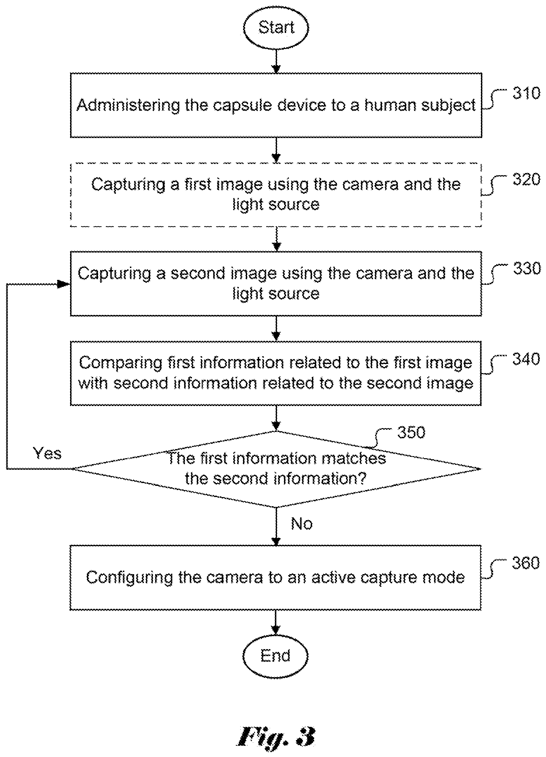

Fig. 3 illustrates an exemplary flowchart of capture control for a capsule device with an enteric coating according to an embodiment of the present disclosure. In step 310, the capsule device is administered to a human subject by swallowing the capsule device. The capsule device is configured to capture a first image using the camera and the light source after said administering the capsule device to the human subject in step 320. The first image should be captured early enough to ensure the coating has not dissolved. The capsule device is configured to capture a second image using the camera and the light source as shown in step 330. The first information related to the first image is then compared with the second information related to the second image in step 340. As mentioned before, the coating image can be captured *in vivo* by the capsule device as shown in step 320. Alternatively, the coating image can be captured off-line using a sample capsule device to derive the first information. In this case, the first information is stored in each capsule device and the step 320 is skipped. The first information is compared to the second information to determine whether the first information matches the second information as shown in step 350. If the first information matches with the second information (i.e., "Yes" path), the capturing process in step 330 and the comparing process in step 340 will be repeated. Otherwise (i.e., "No" path), the coating is determined to has dissolved and the camera is configured to an active capture mode as shown in step 360.

The disclosure may be embodied in other specific forms without departing from its essential characteristics. The described examples are to be considered in all respects only as illustrative and not restrictive. The scope of the invention is, therefore, indicated by the appended claims rather than by the foregoing description. All changes which come within the meaning and range of equivalency of the claims are to be embraced within their scope.

## Claims

1. A method of image capture control for a capsule device (210, 220) with a camera and a light source enclosed in a capsule housing, wherein a coating (212, 222) on outer surface of the housing to cover a field of view of the camera and the coating (212, 222) is dissolvable depending on a factor of an intraluminal environment, **characterized in that** the method comprises:
determining a first image corresponding to a coating image that the capsule device (210, 220) captures with the coating (212, 222) present or a pre-defined image (320);
capturing a second image using the camera and the light source at a first frame rate substantially below a target frame rate (330);
determining whether first information related to the first image matches with second information related to the second image using image processing (340);
if the first information matches with the second information, repeating said capturing the second image and said determining whether the first information related to the first image matches with the second information related to the coating image until the first information does not match with the second information (350); and
if the first information does not match with the second information, declaring that the coating (212, 222) has dissolved and configuring the camera to capture third images at the target frame rate after said declaring that the coating (212, 222) has dissolved (360).

2. The method of Claim 1, wherein said determining whether the first information related to the first image matches with the second information related to the second image is performed using a processing unit inside the capsule device (210, 220), or wherein said determining whether the first information related to the first image matches with the second information related to the second image is performed outside the capsule device (210, 220).

3. The method of Claim 1, wherein said determining the first image corresponds to capturing one initial image using the camera and the light source after the capsule device (210, 220) is administered.

4. The method of Claim 3, wherein the first image is captured at reduced resolution or cropped size or with reduced luminous energy from the light source.

5. The method of Claim 1, wherein the first information and the second information are derived based on partial samples of the first image and the second image respectively.

6. The method of Claim 1, wherein the first information and the second information correspond to color, brightness, or contents of the first image and the second image respectively, and wherein said determining whether the first information related to the first image matches with the second information related to the second image is based on an average or median value of the color or the brightness of the first image and the second image respectively.

7. The method of Claim 1, wherein the first information and the second information are determined based on selected samples at fixed locations of underlying images respectively, and wherein the selected samples are converted to binary data; the selected samples of the first image are compared with the selected samples of the second image on a sample-by-sample basis to obtain a count of matched samples; and the count of matched samples is used to determine whether the coating (212, 222) has dissolved.

8. The method of Claim 1, wherein after said declaring that the coating (212, 222) is dissolved, the camera is configured to wait for a period of time before said capturing the third images at the target frame rate, or wherein the first image is determined using a sample capsule device (210, 220) having a same-type camera coated with a same-type coating and a same-type light source as the capsule device (210, 220), or wherein the capsule device (210, 220) is configured to wait for a period of time before said capturing the first image using the camera and the light source, or wherein the coating image corresponding to the coating (212, 222) includes a blue, green or white area.

9. A capsule device (210, 220) adapted to be swallowed by a patient for imaging gastrointestinal (GI) track of the patient, the capsule device (210, 220) comprising:
an image sensor for capturing images projected on the image sensor;
a light source;
a capsule housing to enclose the image sensor and the light source;
**characterized in that** a coating (212, 222) on outer surface of the capsule housing covering at least an area corresponding a field of view to be imaged by the sensor, wherein the coating (212, 222) is dissolvable depending on a factor of an intraluminal environment; and
a processing unit configured to:
(a) determine a first image corresponding to a coating image that the image sensor captures with the coating (212, 222) present or a pre-defined image (320);
(b) capture a second image using the image sensor and the light source at a first frame rate substantially below a target frame rate (330); and
(c) determine whether first information related to the first image matches with second information related to the second image using image processing (340);
(d) if the first information related to the first image matches with the second information related to the second image, repeat steps (a) to (c) (350); and
(e) if the first information related to the first image does not match with the second information related to the second image, declare that the coating (212, 222) has dissolved and configure the image sensor to capture third images at the target frame rate (360).

10. The capsule device (210,220) of Claim 9, wherein the capsule device (210, 220) further comprising an archival memory to store the third images and the archival memory is enclosed in the capsule housing, or wherein the processing unit is inside the capsule housing.

11. The capsule device (210, 220) of Claim 9, wherein the capsule device (210, 220) further comprising a wireless transmitter inside the capsule housing to transmit the first images to a wireless receiver external to the capsule device (210, 220), and wherein the processing unit is external to the capsule device (210, 220).

12. The capsule device (210, 220) of Claim 9, wherein the coating (212, 222) corresponds to an enteric coating, and wherein a material for the enteric coating is selected from an enteric group consisting of carboxymethylcellulose (CMC), acid substituted cellulose, diacid substituted cellulose such as phthalate and succinate, triacid substituted cellulose such as trimellitic acid and citric acid (CA), acid substituted poly(vinyl alcohol or acetate) (PVA/PVAP), acid-, diacid-, or triacid substituted hydroxypropyl methylcellulose such as phthalic-, succinic-, trimellitic-, and citric-, various substituted methacrylic- and acrylic acid copolymers, fatty acids, waxes, shellac (esters of aleuritic acid (pH 7.0), plastics, and plant fiber.

13. The capsule device (210, 220) of Claim 12, wherein a material for the enteric coating is more specifically selected from an enteric group consisting of carboxymethylcellulose (CMC) (solubility > pH 5.0), cellulose acetate phthalate (CAP) (solubility > pH 6.2), cellulose acetate trimellitate (CAT) (pH 5.0), poly(vinyl acetate phthalate) (PVAP) (> pH 5.2), hydroxypropyl methylcellulose phthalate (HPMCP) (> pH 4.5-5.5), cellulose acetate succinate (solubility pH >5.5-7.0), methacrylic acid copolymer type C/poly(methacrylic acid-co-methyl methacrylate) (pH 5.5-7.0), poly(methyl acrylate-co-methacrylic acid-co-methyl methacrylate) (pH 5.5-7.0), poly(methyl acrylate-co-methacrylic acid-co-ethyl methacrylate), poly(ethyl acrylate-co-methacrylic acid-co-methyl methacrylate), poly(methacrylic acid-co-ethyl methacrylate) fatty acids, waxes, shellac (esters of aleuritic acid) (pH 7.0), plastics, and plant fiber.

14. The capsule device (210, 220) of Claim 9, wherein the coating (212, 222) corresponds to an enzymatic coating, and wherein a material for the enzymatic coating is selected from an enzymatic group consisting of encoded azo crosslinked polymers such as methacrylic acid and/or acrylic acid copolymers, carboxymethylcellulose, hydroxypropylmethylcellulose, methacryloxy azobenzene HEMA, diisocyanate crosslinked dextran.

15. The capsule device (210, 220) of Claim 9, wherein the coating has a double layer structure with inner layer being ionic and outer layer being an enteric coating, wherein water diffuses through the enteric coating into the ionic coating, which absorbs the water and once a sufficient amount of water has been absorbed by the inner layer the outer coating cracks which allow the whole coating matrix to come off, and wherein a material for the coating is selected from a group consisting of hydrophobic surfactant, water-soluble polymer, and hydroxypropyl methylcellulose (HPMC).

## Patentansprüche

1. Verfahren zur Bildaufnahmesteuerung für eine Kapselvorrichtung (210, 220) mit einer Kamera und einer Lichtquelle, die in einem Kapselgehäuse eingeschlossen sind, wobei eine Beschichtung (212, 222) auf einer äußeren Oberfläche des Gehäuses vorgesehen ist, um ein Gesichtfeld der Kamera abzudecken, und die Beschichtung (212, 222) abhängig von einem Faktor einer intraluminalen Umgebung löslich ist, **dadurch gekennzeichnet, dass** das Verfahren umfasst:
Bestimmen eines ersten Bilds entsprechend einem Beschichtungsbild, das die Kapselvorrichtung (210, 220) mit der vorhandenen Beschichtung (212, 222) aufnimmt, oder einem vordefinierten Bild (320);
Aufnehmen eines zweiten Bilds unter Verwendung der Kamera und der Lichtquelle bei einer ersten Framerate im Wesentlichen unterhalb einer Zielframerate (330);
Bestimmen, ob eine erste Information mit Bezug zu dem ersten Bild mit einer zweiten Information mit Bezug zu dem zweiten Bild übereinstimmt, unter Verwendung einer Bildverarbeitung (340);
wenn die erste Information mit der zweiten Information übereinstimmt, Wiederholen der Aufnahme des zweiten Bilds und der Bestimmung, ob die erste Information mit Bezug zu dem ersten Bild mit der zweiten Information mit Bezug zu dem Beschichtungsbild übereinstimmt, bis die erste Information nicht mit der zweiten Information übereinstimmt (350); und
wenn die erste Information nicht mit der zweiten Information übereinstimmt, Festlegen, dass sich die Beschichtung (212, 222) aufgelöst hat, und Konfigurieren der Kamera zum Aufnehmen von dritten Bildern bei der Zielframerate nach der Festlegung, dass sich die Beschichtung (212, 222) aufgelöst hat (360).

2. Verfahren nach Anspruch 1, wobei die Bestimmung, ob die erste Information mit Bezug zu dem ersten Bild mit der zweiten Information mit Bezug zu dem zweiten Bild übereinstimmt, unter Verwendung einer Verarbeitungseinheit innerhalb der Kapselvorrichtung (210, 220) durchgeführt wird, oder wobei die Bestimmung, ob die erste Information mit Bezug zu dem ersten Bild mit der zweiten Information mit Bezug zu dem zweiten Bild übereinstimmt, außerhalb der Kapselvorrichtung (210, 220) durchgeführt wird.

3. Verfahren nach Anspruch 1, wobei die Bestimmung des ersten Bilds einem Aufnehmen eines Anfangsbilds unter Verwendung der Kamera und der Lichtquelle entspricht, nachdem die Kapselvorrichtung (210, 220) verabreicht worden ist.

4. Verfahren nach Anspruch 3, wobei das erste Bild bei reduzierter Auflösung oder abgeschnittener Größe oder mit reduzierter Leuchtenergie von der Lichtquelle aufgenommen wird.

5. Verfahren nach Anspruch 1, wobei die erste Information und die zweite Information basierend auf Teilproben des ersten Bilds beziehungsweise des zweiten Bilds abgeleitet werden.

6. Verfahren nach Anspruch 1, wobei die erste Information und die zweite Information einer Farbe, einer Helligkeit oder Inhalten des ersten Bilds beziehungsweise des zweiten Bilds entsprechen, und wobei die Bestimmung, ob die erste Information mit Bezug zu dem ersten Bild mit der zweiten Information mit Bezug zu dem zweiten Bild übereinstimmt, auf einem Durchschnitts- oder einem Medianwert der Farbe oder der Helligkeit des ersten Bilds beziehungsweise des zweiten Bilds basiert.

7. Verfahren nach Anspruch 1, wobei die erste Information und die zweite Information basierend auf ausgewählten Proben an festen Orten von jeweiligen zugrundeliegenden Bildern bestimmt werden, und wobei die ausgewählten Proben in binäre Daten umgewandelt werden; wobei die ausgewählten Proben des ersten Bilds mit den ausgewählten Proben des zweiten Bilds Probe für Probe verglichen werden, um eine Zahl von übereinstimmenden Proben zu erhalten; und wobei die Zahl von übereinstimmenden Proben dazu verwendet wird zu bestimmen, ob sich die Beschichtung (212, 222) aufgelöst hat.

8. Verfahren nach Anspruch 1, wobei nach der Festlegung, dass sich die Beschichtung (212, 222) aufgelöst hat, die Kamera dazu konfiguriert ist, während einer Zeitperiode vor der Aufnahme der dritten Bilder mit der Zielframerate zu warten, oder wobei das erste Bild bestimmt wird unter Verwendung einer Probenkapselvorrichtung (210, 220) mit einer Kamera vom gleichen Typ, beschichtet mit einer Beschichtung vom gleichen Typ und mit einer Lichtquelle vom gleichen Typ als die Kapselvorrichtung (210, 220), oder wobei die Kapselvorrichtung (210, 220) dazu konfiguriert ist, während einer Zeitperiode vor der Aufnahme des ersten Bilds unter Verwendung der Kamera und der Lichtquelle zu warten, oder wobei das Beschichtungsbild entsprechend der Beschichtung (212, 222) eine blaue, grüne oder weiße Fläche enthält.

9. Kapselvorrichtung (210, 220), die dazu ausgelegt ist, durch einen Patienten für eine Bildgebung des Gastrointestinal-(GI-)Trakts des Patienten geschluckt zu werden, wobei die Kapselvorrichtung (210, 220) umfasst:
einen Bildsensor zum Aufnehmen von Bildern, die auf dem Bildsensor projeziert werden;
eine Lichtquelle;
ein Kapselgehäuse zum Einschließen des Bildsensors und der Lichtquelle;
**dadurch gekennzeichnet, dass** eine Beschichtung (212, 222) auf einer äußeren Oberfläche des Kapselgehäuses wenigstens eine Fläche entsprechend einem Gesichtsfeld bedeckt, das durch den Sensor abzubilden ist, wobei die Beschichtung (212, 222) abhängig von einem Faktor einer intraluminalen Umgebung löslich ist; und
eine Verarbeitungseinheit, die zu Folgendem konfiguriert ist:
(a) Bestimmen eines ersten Bilds entsprechend einem Beschichtungsbild, das der Bildsensor mit der vorhandenen Beschichtung (212, 222) aufnimmt, oder einem vordefinierten Bild (320);
(b) Aufnehmen eines zweiten Bilds unter Verwendung des Bildsensors und der Lichtquelle bei einer ersten Framerate im Wesentlichen unterhalb einer Zielframerate (330); und
(c) Bestimmen, ob eine erste Information mit Bezug zu dem ersten Bild mit einer zweiten Information mit Bezug zu dem zweiten Bild übereinstimmt, unter Verwendung einer Bildverarbeitung (340);
(d) wenn die erste Information mit Bezug zu dem ersten Bild mit der zweiten Information mit Bezug zu dem zweiten Bild übereinstimmt, Wiederholen der Schritte (a) bis (c) (350); und
(e) wenn die erste Information mit Bezug zu dem ersten Bild nicht mit der zweiten Information mit Bezug zu dem zweiten Bild übereinstimmt, Festlegen, dass sich die Beschichtung (212, 222) aufgelöst hat, und Konfigurieren des Bildsensors zum Aufnehmen von dritten Bildern bei der Zielframerate (360).

10. Kapselvorrichtung (210, 220) nach Anspruch 9, wobei die Kapselvorrichtung (210, 220) ferner einen Archivspeicher zum Speichern der dritten Bilder umfasst, und der Archivspeicher in dem Kapselgehäuse eingeschlossen ist, oder wobei die Verarbeitungseinheit innerhalb des Kapselgehäuses ist.

11. Kapselvorrichtung (210, 220) nach Anspruch 9, wobei die Kapselvorrichtung (210, 220) ferner einen drahtlosen Sender innerhalb des Kapselgehäuses umfasst, um die ersten Bilder zu einem drahtlosen Empfänger außerhalb der Kapselvorrichtung (210, 220) zu senden, und wobei die Verarbeitungseinheit außerhalb der Kapselvorrichtung (210, 220) ist.

12. Kapselvorrichtung (210, 220) nach Anspruch 9, wobei die Beschichtung (212, 222) einer enterischen Beschichtung enspricht, und wobei ein Material für die enterische Beschichtung ausgewählt ist aus einer enterischen Gruppe bestehend aus Carboxymethylcellulose (CMC), säuresubstituierter Cellulose, disäuresubstituierter Cellulose wie zum Beispiel Phthalat und Succinat, trisäuresubstituierter Cellulose wie zum Beispiel Trimellitsäure und Zitronensäure (CA), säuresubstituiertem Poly(Vinylalkohol oder Acetat) (PVA/PVAP), säure-, disäure- oder trisäuresubstituierter Hydroxypropylmethylcellulose wie zum Beispiel Phthal-, Bernstein-, Trimellit-, und Zitronen-, verschieden substituierten Methacyl- und Acrylsäurecopolymeren, Fettsäuren, Wachsen, Shellac (Ester von Aleuritinsäure (pH 7.0), Kunststoffen, und Pflanzenfasern.

13. Kapselvorrichtung (210, 220) nach Anspruch 12, wobei ein Material für die enterische Beschichtung spezifischer ausgewählt ist aus einer enterischen Gruppe bestehend aus Carboxymethylcellulose (CMC) (Löslichkeit>pH 5.0), Celluloseacetatphthalat (CAP) (Löslichkeit>pH 6.2), Celluloseacetattrimellitat (CAT) (pH 5.0), Poly(vinylacetatphthalat) (PVAP) (>pH 5.2), Hydroxypropylmethylcellulosephthalat (HPMCO) (>pH 4.5-5.5), Zelluloseacetatsuccinat (Löslichkeit pH>5.5-7.0), Methacylsäurecopolymer-artiges C-Poly(methacrylsäure-co-methylmethacrylat) (pH 5.5-7.0), Poly(methylacrylat-co-methacrylsäure-co-methylmethacrylat) (pH 5.5-7.0), Poly(methylacrylat-co-methacrylsäure-co-ethylmethacrylat), Poly(ethylacrylat-co-methacrylsäure-co-methylmethacrylat), Poly(Methacrylsäure-co-Ethylmethacrylat) Fettsäuren, Wachse, Shellac (Ester von Aleuritinsäure) (pH 7.0), Kunststoffe, und Pflanzenfasern.

14. Kapselvorrichtung (210, 220) nach Anspruch 9, wobei die Beschichtung (212, 222) einer enzymatischen Beschichtung entspricht, und wobei ein Material für die enzymatische Beschichtung ausgewählt ist aus einer enzymatischen Gruppe bestehend aus kodierten azovernetzten Polymeren wie zum Beispiel Methacrylsäure- und/oder Acrylsäure-Copolymere, Carboxymethylcellulose, Hydroxypropylmethylcellulose, Methacryloxyazobenzol-HEMA, Diisocyanatvernetztes Dextran.

15. Kapselvorrichtung (210, 220) nach Anspruch 9, wobei die Beschichtung eine Doppelschichtstruktur mit einer inneren Schicht, die ionisch ist, und einer äußeren Schicht hat, die eine enterische Beschichtung ist, wobei Wasser durch die enterische Beschichtung in die ionische Beschichtung diffundiert, die das Wasser absorbiert, und sobald eine ausreichende Menge von Wasser durch die innere Schicht absorbiert worden ist, die äußere Beschichtung bricht, was es der gesamten Beschichtungsmatrix ermöglicht, sich abzulösen, und wobei ein Material für die Beschichtung ausgewählt ist aus einer Gruppe bestehend aus einem hydrophoben Tensid, einem wasserlöslichen Polymer, und Hydroxypropylmethylcellulose (HPMC).

## Revendications

1. Procédé de contrôle de capture d'images pour un dispositif de capsule (210, 220) avec une caméra et une source de lumière intégrés dans une enveloppe de capsule, qui comporte un enrobage (212, 222) sur une surface externe de l'enveloppe pour couvrir un champ de vision de la caméra et l'enrobage (212, 222) est dissoluble en fonction d'un facteur d'un environnement intraluminal, **caractérisé en ce que** le procédé comporte :
détermination d'une première image correspondant à une image d'enrobage que le dispositif de capsule (210, 220) capture avec l'enrobage (212, 222) présent ou à une image prédéfinie (320),
capture d'une deuxième image en utilisant la caméra et la source de lumière à une première fréquence d'images considérablement en-dessous d'une fréquence d'images visée (330),
détermination de la correspondance entre une première information relative à la première image et une deuxième information relative à la deuxième image par traitement d'images (340),
si la première information correspond à la deuxième information, répétition de ladite capture de la deuxième image et de ladite détermination de la correspondance entre la première information relative à la première image et la deuxième information relative à l'image d'enrobage jusqu'à ce que la première information ne corresponde pas à la deuxième information (350) et
si la première information ne correspond pas à la deuxième information, déclaration que l'enrobage (212, 222) est dissous et configuration de la caméra pour capturer des troisièmes images à la fréquence d'images visée après ladite déclaration que l'enrobage (212, 222) est dissous (360).

2. Procédé selon la revendication 1, dans lequel ladite détermination de la correspondance entre la première information relative à la première image et la deuxième information relative à la deuxième image est réalisée en utilisant une unité de traitement à l'intérieur du dispositif de capsule (210, 220), ou dans lequel ladite détermination de la correspondance entre la première information relative à la première image et la deuxième information relative à la deuxième image est réalisée à l'extérieur du dispositif de capsule (210, 220).

3. Procédé selon la revendication 1, dans lequel ladite détermination de la première image correspond à la capture d'une image initiale en utilisant la caméra et la source de lumière après l'administration du dispositif de capsule (210, 220).

4. Procédé selon la revendication 3, dans lequel la première image est capturée à une résolution réduite ou à une taille rognée ou avec une énergie lumineuse de la source de lumière réduite.

5. Procédé selon la revendication 1, dans lequel la première information et la deuxième information sont dérivées, sur la base d'échantillons partiels de la première image et de la deuxième image respectivement.

6. Procédé selon la revendication 1, dans lequel la première information et la deuxième information correspondent à la couleur, la luminosité ou des contenus de la première image et de la deuxième image respectivement, et dans lequel ladite détermination de la correspondance entre la première information relative à la première image et la deuxième information relative à la deuxième image est basée sur une valeur moyenne ou médiane de la couleur ou de la luminosité de la première image et de la deuxième image respectivement.

7. Procédé selon la revendication 1, dans lequel la première information et la deuxième information sont déterminées, sur la base d'échantillons sélectionnés à des emplacements fixes d'images sous-jacentes respectivement, et dans lequel les échantillons sélectionnés sont convertis en données binaires ; les échantillons sélectionnés de la première image sont comparés aux échantillons sélectionnés de la deuxième image, un échantillon à la fois, pour obtenir un décompte d'échantillons correspondants ; et le décompte d'échantillons correspondants est utilisé pour déterminer si l'enrobage (212, 222) est dissous.

8. Procédé selon la revendication 1, dans lequel après ladite déclaration que l'enrobage (212, 222) est dissous, la caméra est configurée pour attendre un laps de temps avant de capturer les troisièmes images à la fréquence d'images visée, ou dans lequel la première image est déterminée en utilisant un dispositif de capsule échantillon (210, 220) avec une caméra du même type revêtue d'un enrobage du même type et une source de lumière du même type que le dispositif de capsule (210, 220), ou dans lequel le dispositif de capsule (210, 220) est configuré pour attendre un laps de temps avant ladite capture de la première image en utilisant la caméra et la source de lumière, ou dans lequel l'image d'enrobage correspondant à l'enrobage (212, 222) inclut une zone bleue, verte ou blanche.

9. Dispositif de capsule (210, 220) adapté pour être avalé par un patient pour capturer le parcours gastro-intestinal (GI) du patient, le dispositif de capsule (210, 220) comprenant :
un capteur d'images pour capturer des images projetées sur le capteur d'images,
une source de lumière,
une enveloppe de capsule pour intégrer le capteur d'images et la source de lumière,
**caractérisé en ce qu'**un enrobage (212, 222) sur la surface externe de l'enveloppe de la capsule couvre au moins une zone correspondant à un champ de vision à capturer par le capteur, dans lequel l'enrobage (212, 222) est dissoluble en fonction d'un facteur d'un environnement intraluminal, et
une unité de traitement configurée pour :
(a) déterminer une première image correspondant à une image d'enrobage que le capteur d'images capture avec l'enrobage (212, 222) présent ou à une image prédéfinie (320),
(b) capturer une deuxième image en utilisant le capteur d'images et la source de lumière à une première fréquence d'images considérablement en-dessous de la fréquence d'images visée (330), et
(c) déterminer si une première information relative à la première image correspond à une deuxième information relative à la deuxième image en utilisant un traitement d'images (340),
(d) si la première information relative à la première image correspond à la deuxième information relative à la deuxième image, répéter les étapes (a) à (c) (350), et
(e) si la première information relative à la première image ne correspond pas à la deuxième information relative à la deuxième image, déclarer que l'enrobage (212, 222) est dissous et configurer le capteur d'images pour capturer des troisièmes images à la fréquence d'images visée (360).

10. Dispositif de capsule (210, 220) selon la revendication 9, dans lequel le dispositif de capsule (210, 220) comprend également une mémoire d'archives pour enregistrer les troisièmes images et la mémoire d'archives est intégrée dans l'enveloppe de la capsule, ou dans lequel l'unité de traitement est à l'intérieur de l'enveloppe de la capsule.

11. Dispositif de capsule (210, 220) selon la revendication 9, dans lequel le dispositif de capsule (210, 220) comprend également un émetteur sans fil à l'intérieur de l'enveloppe de la capsule pour transmettre les premières images à un récepteur sans fil externe au dispositif de capsule (210, 220), et dans lequel l'unité de traitement est externe au dispositif de capsule (210, 220).

12. Dispositif de capsule (210, 220) selon la revendication 9, dans lequel l'enrobage (212, 222) correspond à un enrobage entérique, et dans lequel un matériau pour l'enrobage entérique est choisi dans un groupe entérique composé de carboxyméthylcellulose (CMC), d'acétate de cellulose, de diacétate de cellulose tel que le phtalate et le succinate, de triacétate de cellulose tel que l'acide trimellitique et l'acide citrique (CA), d'acétate de poly(alcool vinylique ou acétate de vinyle) (PVA/PVAP), d'acétate, de diacétate ou de triacétate d'hydroxypropylméthylcellulose tel que des copolymères d'acide acrylique et de plusieurs acides méthacryliques substitués, d'acide succinique, trimellitique et citrique, d'acides gras, de cires, de laque (esters d'acide aleuritique) (pH 7.0), de plastiques et de fibres végétales.

13. Dispositif de capsule (210, 220) selon la revendication 12, dans lequel un matériau pour l'enrobage entérique est choisi plus spécifiquement dans un groupe entérique composé de carboxyméthylcellulose (CMC) (solubilité > pH 5.0), d'acétate et phtalate de cellulose (CAP) (solubilité > pH 6.2), d'acétate de cellulose trimellitate (CAT) (pH 5.0), de poly(acétate phtalate de vinyle) (PVAP) (> pH 5.2), de phtalate d'hydroxypropylméthylcellulose (HPMCP) (> pH 4.5-5.5), d'acéto-succinate de cellulose (solubilité pH >5.5-7.0), de copolymère d'acide méthacrylique de type C/poly(méthacrylate de méthyle-co-acide méthacrylique) (pH 5.5-7.0), poly(méthacrylate de méthyle-co-acide méthacrylique-co-acrylate de méthyle) (pH 5.5-7.0), poly(méthacrylate d'éthyle-co-acide méthacrylique-co-acrylate de méthyle), poly(méthacrylate de méthyle-co-acide méthacrylique-co-acrylate d'éthyle), poly(méthacrylate d'éthyle-co-acide méthacrylique), d'acides gras, de cires, de laque (esters d'acide aleuritique) (pH 7.0), de plastiques et de fibres végétales.

14. Dispositif de capsule (210, 220) selon la revendication 9, dans lequel l'enrobage (212, 222) correspond à un enrobage enzymatique, et dans lequel un matériau pour l'enrobage enzymatique est choisi dans un groupe enzymatique composé de polymères encodés azo-réticulés tels que de l'acide méthacrylique et/ou des copolymères d'acide acrylique, de carboxyméthylcellulose, d'hydroxypropylméthylcellulose, d'azobenzène méthacryloxy HEMA, de dextrane réticulé avec un diisocyanate.

15. Dispositif de capsule (210, 220) selon la revendication 9, dans lequel l'enrobage a une structure à double couche, dont une couche interne est ionique et une couche externe est un enrobage entérique, dans lequel de l'eau se diffuse à travers l'enrobage entérique dans l'enrobage ionique, qui absorbe l'eau et une fois qu'une quantité suffisante d'eau a été absorbée par la couche interne, l'enrobage externe se fissure, ce qui permet à toute la matrice de l'enrobage de se détacher, et dans lequel un matériau pour l'enrobage est choisi dans un groupe composé d'agent tensioactif hydrophobe, de polymère hydrosoluble, et d'hydroxypropylméthylcellulose (HPMC).
